## Europäisches Patentamt
## European Patent Office
## Office européen des brevets

(19)

(11) Veröffentlichungsnummer : **0 110 193**
**B1**

(12)
# EUROPÄISCHE PATENTSCHRIFT

(45) Veröffentlichungstag der Patentschrift :
09.09.87

(51) Int. Cl.⁴ : **B 22 C  7/02**, B 22 C  9/04,
A 61 C  5/10, A 61 C 13/00

(21) Anmeldenummer : **83111044.0**

(22) Anmeldetag : **04.11.83**

(54) **Verfahren zur Herstellung individueller Gussteile.**

(30) Priorität : **05.11.82 DE 3240907**

(43) Veröffentlichungstag der Anmeldung :
**13.06.84 Patentblatt 84/24**

(45) Bekanntmachung des Hinweises auf die Patenterteilung : **09.09.87 Patentblatt 87/37**

(84) Benannte Vertragsstaaten :
**AT BE CH DE FR GB IT LI LU NL SE**

(56) Entgegenhaltungen :
**DE-A- 2 910 077
DE-A- 2 914 537
US-A- 4 249 591
US-A- 4 347 888
Lexikon der. Zahnmedizin, 1978, S. 243 (Verl. "Die
Quintessenz")**

(73) Patentinhaber : **ESPE Stiftung & Co Produktions- und
Vertriebs KG**

**D-8031 Seefeld (DE)**

(72) Erfinder : **Pfannenstiel, Hubert
Tiefenweg 5
D-8712 Lenggries (DE)**
Erfinder : **Hübner, Heijo, Dr.
Moosbichlweg 16
D-8031 Wörthsee (DE)**

(74) Vertreter : **Müller, Bernhard, Dr.
Graf-Toerring-Strasse 45
D-8031 Seefeld 2 (DE)**

Jouve, 18, rue St-Denis, 75001 Paris, France

## Beschreibung

Die Erfindung betrifft ein Verfahren zur Herstellung von individuellen Gußteilen, zum Beispiel auf dem Gebiet der Dentaltechnik oder der Schmuckherstellung, gemäß dem Oberbegriff des Anspruchs 1.

Zur bekannten Herstellung von prothetischen Metallgerüsten in der Dentaltechnik, wie z. B. von Kronen, Inlays oder Brücken, werden diese zuerst in Wachs modelliert und dadurch mit der entsprechenden individuellen Formgebung versehen. An diesem sogenannten Gußmodell wird ein vorgefertigter Gußkanal befestigt ; anschließend wird das Gußmodell mit einer feuerfesten Einbettmasse umkleidet. Die Wachsmasse kann nun entweder durch Ausbrühen mit heißem Wasser oder durch Verbrennen des Wachses bei Temperaturen zwischen ca. 500 und 700 °C entfernt werden. In die entstandene Negativform wird dann flüssiges Metall gebracht, man läßt abkühlen, entformt und erhält so ein dem Gußmodell aus Wachs formgleiches Gußteil. Der vorstehend geschilderte Stand der Technik ergibt sich beispielsweise aus Lexikon der Zahnmedizin, Buch- und Zeitschriftverlag « Die Quintessenz », Berlin 1978, S. 243, Stichwort « Gußverfahren ».

Es ist offensichtlich, daß das Gußteil hinsichtlich seiner Paßgenauigkeit in der entsprechenden Situation im Munde nur so gut ist wie das Gußmodell aus Wachs. Üblicherweise verfügt der Zahntechniker über ein Kiefermodell z. B. aus Gips, an welches er das Gußmodell anpaßt. Beim Abheben der in Wachs modellierten Teile vom Modell besteht jedoch eine große Gefahr, daß das Gußmodell sich verzieht und damit auch der letztlich erhaltene Metallguß nicht mehr paßt. Das Wachs muß außerdem beim Modellieren durch Erhitzen verflüssigt werden, so daß besonders bei größeren Brückenkonstruktionen starke thermische Spannungen entstehen, die zu einem Verziehen oder Brechen des Gußmodells führen können. Durch ungleichmäßige Temperatur erhält man auch unterschiedliche Wachskonsistenzen, so daß die Gleichmäßigkeit des Wachsauftrags nicht immer gewährleistet ist und sich die Modellierung insgesamt schwierig gestaltet. Eine zusätzliche Gefahr liegt in der Bildung von Schlieren und Bläschen.

Um der Verformung des Wachses beim Abheben vom Modell entgegenzuwirken, ist man dazu übergegangen, über Kronenstümpfe Käppchen aus Kunststofftiefziehfolie herzustellen. Durch die anschließende thermische Belastung beim Auftragen des geschmolzenen Wachses besteht jedoch erneut die Gefahr einer Verformung. Besonders schwierig ist die Situation bei mehrteiligen, abnehmbaren Konstruktionen, wie z. B. Konuskronen, Teleskopkronen oder Doppelkronen für Verriegelungen. Diese Konstruktionen bestehen prinzipiell aus einer als Primärteil bezeichneten und auf dem Zahnstumpf befestigbaren Metallkrone, über welche eine sogenannte Sekundärkrone paßgenau angefertigt wird. An diesses Sekundärteil werden später Prothesen oder Brücken befestigt, die damit abnehmbar sind. Voraussetzung für die Erfüllung der Haltefunktion von Sekundärteilen auf Primärteilen ist die paßgenaue Herstellung der Sekundärkrone auf der Primärkrone. Ähnliches gilt für Geschiebe-, Riegel- und Stegkonstruktionen. Da es sehr schwierig ist, diese ineinandergreifenden Teile aus Wachs zu modellieren, werden zur Erhöhung der Stabilität hauptsächlich die Sekundärteile dieser abnehmbaren Prothesen mit autopolymerisierendem Methylmethacrylat zumindest in einer gewissen Schichtdicke modelliert. Ein derartiges Verfahren ist beschrieben in « Quintessenz » 19-65, Heft 6, S. 57-58.

Diese meist nach dem Prinzip der Redoxpolymerisation aushärtenden Massen haben jedoch eine relativ kurze Verarbeitungsbreite und brauchen dennoch verhältnismäßig lange, bis sie auspolymerisiert sind. Durch die hohe Temperaturspitze bei der Aushärtung kommt es gerne zu thermischen Spannungen, außerdem bilden sich leicht Bläschen an der Oberfläche. Durch die hohe Polymerisationsschrumpfung ist es weiterhin notwendig, die Masse in größerer Menge aufzutragen und sie dann auf das endgültige Maß zurückzuschleifen. Dieser Umstand verhindert eine genaue anatomische Modellation im aufbauenden Verfahren und macht sie daher außerordentlich umständlich und zeitraubend. Dies wird auch dadurch bedingt, daß die Polymerisation gleich nach dem Anmischen der Masse einsetzt und sich die Konsistenz der Masse während der Arbeit kontinuierlich verändert.

Am Anfang der Arbeit bleibt die Methylmethacrylatzubereitung noch relativ lange flüssig, so daß die Partien, die nicht abgedeckt werden sollen, umständlich z. B. durch einen Wachsring vor einem Überlaufen geschützt werden müssen. Das Arbeiten mit diesen Polymerisaten ist also ein sehr aufwendiges und umständliches Verfahren, das zudem überdurchschnittlich hohen Materialverbrauch mit sich bringt.

Aufgabe der Erfindung ist daher die Bereitstellung eines Verfahrens, das die einfache Herstellung paßgenauer Gußmodelle auch bei schwierigsten Konstruktionen, besonders in der Dentaltechnik, ermöglicht.

Diese Aufgabe wird erfindungsgemäß gelöst durch die Schaffung eines Verfahrens zur Herstellung individueller Metallgußteile, mittels eines Gussmodells, in welchem man

am Gußmodell mindestens einen Gießkanal befestigt,

das Gußmodell in eine feuerfeste Formmasse einbettet,

das Gußmodell durch Erhitzen rückstandsfrei entfernt,

in die so erhaltene Form flüssiges Metall einbringt,

die Form erkalten läßt und

das Gußteil ausbettet,
das dadurch gekennzeichnet ist, dass man das Gussmodell mindestens teilweise durch Photopolymerisation einer photopolymerisierbaren Masse, enthaltend di- oder polyfunktionelle (Meth)Acrylsäureester, aufbaut.

In der DE-OS 2 910 077 ist die Herstellung von Zahnprothesen durch Photopolymerisation von Kunststoffen beschrieben. Ein Hinweis auf dei Herstellung von Gußmodellen für Gießarbeiten taucht nirgends auf.

Zur Durchführung des erfindungsgemäßen Verfahrens wird eine rasch photopolymerisierbare Substanz zusammen mit einem Photoinitiator verwendet. Es ist offensichtlich, daß der durch Photopolymerisation aus diesen Zubereitungen erhaltene Kunststoff z. B. durch Verbrennen rückstandsfrei entfernbar sein muß.

Die erfindungsgemäss verwendeten di- oder polyfunktionellen (Meth)Acrylsäureester führen zu besonders verformungsstabilen Gussmodellen.

Es können die bekannten Photoinitiatoren eingesetzt werden, wie z. B. Benzophenone, Benzoinether, Benzilketale, Thioxanthone und aromatische oder aliphatische Diketone. Besonders geeignet sind 1,2-Diketone, insbesondere Campherchinon und Phenanthrenchinon.

Darüber hinaus können sogenannte Photoaktivatoren verwendet werden, wie z. B. organische Phosphite oder Amine. Im Falle der 1,2-Diketone sind tertiäre Amine besonders wirksame Aktivatoren.

Zur Erhöhung der mechanischen Festigkeit, Reduzierung des Polymerisationsschrumpfes und zur Kontrolle der Viskosität können die Zubereitungen zur Durchführung des erfindungsgemäßen Verfahrens auch organische Füllstoffe und/oder lösliche organische Kunststoffe enthalten. Geeignet sind z. B. Polymethylmethacrylat, Nylonperlen, Polyvinylacetat, Polyvinylchlorid oder Polystyrol. Es muß jedoch gewährleistet sein, daß die ausgewählten Zusätze rückstandsfrei verbrennen oder in der Hitze ausgeschmolzen werden.

Die Viskosität der erfindungsgemäßen Zubereitung wird in günstiger Weise durch die genannten Maßnahmen so eingestellt, daß die Masse mit einem Pinsel oder Spatel dünn aufzutragen ist, aber ohne Einwirkung von Scherkräften nur ein geringes Fließvermögen aufweist.

Zur besseren Unterscheidung vom Kiefermodell und zur Kontrolle der aufgetragenen Schichtstärke ist es vorteilhaft, die verwendete, photopolymerisierbare Zubereitung mit Pigmenten und/oder Trübungsmitteln zu versehen. Besonders günstig kann die Masse verarbeitet werden, wenn sie eine geringe Transparenz aufweist, denn dann kann vom Verarbeiter die aufgetragene Schichtstärke anhand des Opazitätsgrades mit dem Auge kontrolliert werden. Wiederum ist es von Bedeutung, daß die ausgewählten Zusätze rückstandsfrei entfernbar sind.

Die Wellenlänge des bei der Photopolymerisation der beschriebenen Zubereitungen verwendeten Lichts muß so ausgewählt werden, daß sie der wirksamen Lichtabsorption des Photoinitiators entspricht. Wegen der Unschädlichkeit der Strahlung und der einfachen und preisgünstigen Konstruktion der Bestrahlungseinheit ist es vorteilhaft, sichtbares Licht im Wellenlängenbereich von ca. 400-500 nm, bevorzugt zusammen mit 1,2-Diketon-Initiatoren zu verwenden.

Natürlich sind auch die kürzerwelligen Strahlungsbereiche ebenfalls verwendbar, wenn für geeignete Schutzmaßnahmen gesorgt wird.

Die Massen, welche außerdem noch die üblichen Stabilisatoren und Inhibitoren enthalten können, werden in lichtdichten Behältern zur Verwendung bereitgehalten.

Nach dem erfindungsgemäßen Verfahren können die oben beschriebenen photopolymerisierbaren Zubereitungen bei der Herstellung von Gußmodellen das Wachs ganz oder teilweise ersetzen. Das lichthärtende Material wird mit einem Pinsel auf den Zahnstumpf oder auf das entsprechende Primärteil in dünner Schicht aufgetragen, und diese dann durch Belichtung mit dem entsprechenden Licht in einigen Sekunden oder Minuten ausgehärtet. Nach diesem Verfahren kann entweder eine formstabile Basis aus photopolymerisiertem Kunststoff hergestellt werden, der weitere Aufbau erfolgt dann wie üblich mit Wachs. Durch schrittweisen Aufbau in dünnen Schichten kann aber auch das gesamte Wachs durch das Photopolymerisat ersetzt werden. Dadurch, daß die Schichten unter Lichteinwirkung sofort zu einem harten, formstabilen Kunststoff erhärten, ist ein Verziehen oder Wegfließen des Materials nicht mehr möglich. Gegebenenfalls muß vor der Anwendung des lichthärtenden Kunststoffs das Gipsmodell oder das Primärteil mit einem geeigneten Isoliermittel (z. B. Silikon) bedeckt werden.

Durch die lange Verarbeitungszeit in Abwesenheit einer intensiven Lichtquelle ist das Auftragen in kleinen Portionen und gezielte Modellieren der entsprechenden Teile möglich. Dadurch entfallen aufwendige Korrekturarbeiten durch Schleifen oder ähnliches. Ein umständliches Abdecken der Teile, die mit dem Material nicht in Berührung kommen sollen, z. B. mit Wachs ist nicht notwendig, da ein schnelles Fixieren und Stabilisieren der aufgetragenen Masse möglich ist. Das stabile Polymerisat ermöglicht ein Lösen der hergestellten Gußmodelle auch im halbfertigen Zustand, um z. B. die Innenflächen zu kontrollieren. Weiterhin wird durch das Auftragen und Fixieren in kleinen Portionen eine zu starke Erwärmung und damit Bläschenbildung oder Schlieren zuverlässig vermieden.

Da die Aushärtung des Modelliermaterials durch gezieltes und beeinflußbares Bestrahlen erfolgt, ist ein besonders sorgfältiges und rationelles Arbeiten mit den Massen möglich ; große Materialüberschüsse sind nicht zu verwerfen, da nur die benötigte Menge Material der Packung entnommen wird. Durch das Auftragen in dünnen Schichten wird auch die Polymerisationsschrumpfung in großem Umfang kompensiert. Nach dem Auftragen jeder Schicht wird durch Bestrahlen mit einer intensiven Lichtquelle ausgehärtet.

Nach Fertigstellung des Gußmodells durch Bestrahlung der photopolymerisierbaren Zubereitung und gegebenenfalls weitere Ergänzung mit Wachs wird wie üblich ein Gußkanal angebracht. Um eine leichtere Entfernung des erhaltenen Gußmodells durch Ausschmelzen und/oder Verbrennen in der Hitze zu ermöglichen, kann es günstig sein, neben dem Hauptgußkanal weitere Kanäle am Gußmodell anzubringen. Dann wird das Gußmodell mit einer Einbettmasse umkleidet. Bei einer Temperatur von ca. 500-700 °C wird das Gußmodell rückstandsfrei durch Ausschmelzen und/oder Verbrennen entfernt und anschließend wie üblich das flüssige Metall eingebracht.

Auch die Befestigung von Teilen, die verlötet werden sollen, wie z. B. Kronen, Brücken oder Klammern, ist statt mit Klebewachs mit dem lichthärtenden Werkstoff möglich. Die Befestigung erfolgt nach Fixierung der Teile auf dem Modell durch Photopolymerisation. Anschließend wird wie üblich teilweise mit Löteinbettmasse ummantelt, der Kunststoff vom Metall abgebrannt und verlötet.

Im Folgenden wird das erfindungsgemäße Verfahren anhand von Beispielen aus der dentalen Technik näher erläutert. Das erfindungsgemäße Verfahren ist jedoch nicht auf die Dentaltechnik beschränkt, sondern überall dort anwendbar, wo hochwertige, individuelle Gußteile kleinerer Dimension gefertigt werden. Anwendungsmöglichkeiten sind also beispielsweise auch gegeben bei individuellen Gußteilen für hochwertige Maschinen, in der Schmucktechnik und in der orthopädischen Prothetik z. B. bei Gußteilen zur Ausfüllung von Knochendefekten.

Beispiel 1

(Herstellung eines Inlays)

Der positive Modellstumpf der präparierten Kavität wird zunächst mit einer Isolierlösung auf Alginatbasis behandelt. Eine Mischung aus 4 Gewichtsteilen Bis-GMA (Reaktionsprodukt aus Bisphenol A und 2 Äquivalenten Glydicylmethacrylat) und einem Gewichtsteil Triglykoldimethacrylat, initiiert mit 0,2 % Campherchinon und 1,5 % Triethanolamin, wird mit einem Pinsel in dünnen Schichten aufgetragen und anschließend jeweils 10 Sekunden mit einem handelsüblichen, dentalen Bestrahlungsgerät für sichtbares Licht (Elipar, Firma ESPE) bestrahlt. Wenn das Gußmodell fertig modelliert ist, wird es aus der Kavität entnommen, ein vorgefertigter Gußkanal aus Wachs daran befestigt und in eine feuerfeste Masse eingebettet. Durch Erhitzen auf ca. 600 °C wird das Gußmodell rückstandsfrei verbrannt und anschließend auf übliche Weise flüssiges Metall (Goldlegierung) in die erhaltene Gußform eingebracht. Nach dem Erkalten wird wie üblich ausgebettet und ausgearbeitet. Man erhält ein höchst paßgenaues Goldgußinlay.

Beispiel 2

(Herstellung einer Krone)

Der Modellstumpf wird mit einem üblichen, feuchtigkeitsvulkanisierenden Silikonfilm isoliert. Wie in Beispiel 1 beschrieben, wird nun durch schichtweises Auftragen der lichtpolymerisierbaren Masse, die jedoch statt Triglykoldimethacrylat Bishydroxymethylbicyclo [5.2.1.0$^{2,6}$]-decandiacrylat enthält, und jeweiliges Belichten ein gleichmäßiges Käppchen von etwa 0,5 mm Wandstärke über den Stumpf gezogen. Das erhärtete Käppchen ist ohne Formänderung vom Stumpf abzunehmen, z. B. zur Kontrolle der Innenflächen und der Ränder.

Auf das Käppchen kann nun wie üblich mit Wachs, oder entsprechend Beispiel 1 durch schichtweises Auftragen und Belichten der lichthärtenden Masse die Krone modelliert werden. Anschließend wird wie üblich mit Gußkanal versehen, eingebettet, Kunststoff und Wachs bei ca. 600 °C verbrannt und gegossen. Man erhält eine paßgenaue Krone.

Beispiel 3

(Herstellung einer Brücke)

Zur Herstellung einer Brücke werden die Modellstümpfe wie üblich isoliert und wie in Beispiel 2 beschrieben mit einem Käppchen von 0,5 mm Wandstärke mit photogehärtetem Kunststoff beschichtet. Der Photoinitiator ist in diesem Falle statt Campherchinon Benzil. Die Käppchen der einzelnen Stümpfe werden nun durch Auftragen des photopolymerisierenden Kunststoffes und Belichten mit einem stabilisierenden Gerüst verbunden, der weitere Aufbau kann nun wiederum wie üblich mit Wachs oder mit photopolymerisierbarer Masse geschehen. Der weitere Guß erfolgt wie üblich. Die erhaltene Brücke ist spannungsfrei und paßt genau auf das Modell.

Beispiel 4

(Herstellung des Sekundärteils einer Konuskrone)

Zur Herstellung einer herausnehmbaren Brücke mit einer Konuskrone wird wie üblich die in Metall gefräste fertige Primärkrone vom Zahnstumpf abgenommen und auf der Außenfläche wie in Beispiel 2 beschrieben mit einer gleichmäßigen Schicht von ca. 0,5 mm des lichthärtenden Materials ummantelt. Nach der Bestrahlung kann die Kunststoffkappe zur Überprüfung der Innenseite von der Primärkrone abgenommen werden. Nach der Überprüfung wird die entstandene paßgenaue Hülse wieder auf das Primärteil aufgesetzt und die anatomische Form der Sekundärkrone entweder in Wachs modelliert oder durch portionsweises Auftragen und Härten des lichthärtenden Kunststoffes modellierend aufgebaut.

Die modellierte und ausgehärtete Sekundärkrone wird dann von der Primärkrone abgenommen und wie üblich mit einem Gußstift aus Wachs versehen und mit feuerfester Einbettmasse umkleidet. Durch Erhitzen auf ca. 700 °C werden Wachs und lichthärtender Kunststoff rückstandslos verbrannt. Wie üblich wird dann gegossen, ausgebettet und ausgearbeitet. Man erhält eine Sekundärkrone aus Metall, die in Form und Paßgenauigkeit mit der Sekundärkrone aus lichthärtendem Material identisch ist und gute Konushaftung auf der primärkrone aufweist.

In ähnlicher Weise können auch die Sekundärteile von Teleskopkronen und Doppelkronen einschließlich der Verriegelungselemente (z. B. Dreh-, Schwenk- oder Steckriegel) hergestellt werden.

Beispiel 5

(Herstellung von Patrizenteilen für individuelle Geschiebe)

Die schwalbenschwanzförmig gefräste Aussparung einer Vollgußkrone (Matrize) wird mit einem Gemisch von 7 Gewichtsteilen des Reaktionsprodukts aus 2,2,4-Trimethyl-hexamethylen-diisocyanat und 2 Äquivalenten Hydroxyethyl-methacrylat, mit einem gewichtsteil Triglykoldimethacrylat, das 0,2 % Campherchinon und 1 % Methyldiethanolamin enthält, gefüllt und durch eine 40-Sekunden Belichtung mit einem dentalen Bestrahlungsgerät ausgehärtet. Anschließend wird wie in Beispiel 2 beschrieben die Außenform des Patrizenteils schichtweise aufgebaut. Nach dem Härten wird das Teil aus lichtgehärtetem Material von der Matrize entfernt. Die entstandene Patrize wird dann wie üblich gegossen. Man erhält ein Patrizenteil, das problemlos in die vogegebene, gefräste Matrize paßt.

Da individuelle Geschiebe häufig in Verbindung mit Kronen und Brücken verwendet werden, die sowohl die Matrize als auch die Patrize tragen, ist es möglich, die Patrizenteile an die in Wachs modellierten Kronen oder Brücken anzuwachsen und mitzugießen.

Natürlich können auch Patrizenteile für individuelle Geschiebe nach dem beschriebenen, anmeldungsgemäßen Verfahren an Verblendkronen und Brücken aus Metall hergestellt werden ; die Aussparungen können auch T-nutförmig oder rillenförmig sein. Die erhaltenen Patrizenteile in Metall haben hohe Paßgenauigkeit und erfüllen dadurch voll ihre Haltefunktion durch Friktion und ihre Rolle als Führung beim Abnehmen und Einsetzen.

Beispiel 6

(Herstellung eines teleskopierenden Ankers)

Auch beim teleskopierenden Anker wird das Sekundärteil durch friktive Gleitreibung auf dem Primärteil geführt und gehalten, so daß es hohe Paßgenauigkeit aufweisen muß. Es wird jedoch nicht die gesamte Außenfläche der Primärkronenfläche gefräst und als Funktionsfläche verwendet, sondern nur ein Teil. Der verbleibende Restteil der primärkrone wird entweder in seiner anatomischen Form in Metall belassen, oder aber nach entsprechender Gestaltung mit einem Verblendmaterial versehen. Im gefrästen Anteil der Primärkrone werden Rillen und Schultern eingefräst, die zur Führung und Abstützung des Sekundäranteils auf dem Primärteil dienen. Diese Rillen und Schultern im Sekundärteil exakt wiederzugeben, ist besonders schwierig ; außerdem darf der ungefräste Teil der Sekundärkrone nicht mit dem Gußmodellmaterial in Berührung kommen.

Analog wie in Beispiel 4 beschrieben wird die Sekundärkrone auf der gefrästen Außenseite der Primärkrone aus der lichthärtenden Masse schichtweise aufgebaut und nach jeder Schicht durch Bestrahlung gehärtet. Auf diese Weise ist eine Abgrenzung der ungefrästen Teile, die nicht mit Kunststoff bedeckt werden sollen (z. B. mit Wachs) nicht erforderlich. Man erhält auf diese Weise ein spannungsfreies Sekundärteil, das ohne Schlierenbildung an der Oberfläche hergestellt wird. Anschließend wird wie üblich eingebettet, das Gußmodell bei 700 °C verbrannt und dann gegossen. Man erhält so auf einfache Weise einen paßgenauen teleskopierenden Anker.

In ähnlicher Weise kann auch ein Stegüberwurf, in diesem Falle das Sekundärteil, über dem Primärteil Steg hergestellt werden.

Beispiel 7

(Vorbereitende Verbindung zum Verlöten)

Eine Brücke und die dazugehörige Klammer werden auf das Gipsmodell gegeben und dort durch Auftragen des lichtpolymerisierbaren Kunststoffs und anschließende Aushärtung durch Bestrahlung aneinander befestigt. Anschließend wird der Verband vom Modell abgehoben, mit einer feuerfesten Masse teilweise umkleidet und so in der Position weiter fixiert. Wie üblich wird nach dem Aushärten der Löteinbettmasse der photogehärtete Kunststoff von den Metallteilen weggebrannt ; anschließend wird in üblicher Weise verlötet. Die erhaltene Verbindung ist höchst paßgenau auf dem vorliegenden Modell.

**Patentansprüche**

1. Verfahren zur Herstellung individueller Metallgußteile mittels eines Gußmodells, in welchem man

am Gußmodell mindestens einen Gießkanal befestigt,

das Gußmodell in eine feuerfeste Formmasse einbettet,

das Gußmodell durch Erhitzen rückstandsfrei entfernt,

in die so erhaltene Form flüssiges Metall einbringt,

die Form erkalten läßt und

das Gußteil ausbettet,
das dadurch gekennzeichnet ist, dass man das Gussmodell mindestens teilweise durch Photopolymerisation einer photopolymerisierbaren Masse, enthaltend di- oder polyfunktionelle (Meth)Acrylsäureester, aufbaut.

2. Verfahren nach Anspruch 1, dadurch gekennzeichnet, daß die Photopolymerisation im Wellenlängenbereich von 400-500 nm durchgeführt wird.

3. Verfahren nach Anspruch 2, dadurch gekennzeichnet, daß man zur Photopolymerisation Campherchinon als Photoinitiator verwendet.

4. Verfahren nach Anspruch 1 bis 3, dadurch gekennzeichnet, daß die photopolymerisierbare Masse organische Pigmente und/oder Trübungsmittel enthält.

5. Verfahren nach Anspruch 1 bis 4, dadurch gekennzeichnet, daß die photopolymerisierbare Masse in mehreren Schichten aufgetragen und jede Schicht einzeln der Photohärtung unterzogen wird.

6. Verfahren nach Anspruch 1 bis 5, dadurch gekennzeichnet, daß man als Gußmodell in der Dentaltechnik eine Krone, ein Inlay, eine Brücke, das Sekundärteil einer Konuskrone, Teleskopkrone, Doppelkrone oder ein Patrizenteil für individuelle Geschiebe sowie einen teleskopierenden Anker oder einen Stegüberwurf oder eine Lötverbindung aufbaut.

## Claims

1. A method of producing individual metal castings using a casting model wherein,
at least one sprue is secured to the casting model,
the casting model is embedded in a refractory moulding compound,
the casting model is removed without residue by heating,
molten metal is introduced into the mould obtained,
the mould is allowed to cool and
the casting is removed from the mould.
characterized in that the casting model is built up at least partially by photopolymerization of a photopolymerizable compound containing di- or polyfunctional (meth)acrylic acid esters.

2. A method according to claim 1, characterized in that the photopolymerization is carried out in the wavelenght range of 400-500 nm.

3. A method according to claim 2, characterized in that the photoinitiator is camphor quinone.

4. A method according to claims 1 an 3, characterized in that the photopolymerizable compound contains organic pigments and/or opacifying agents.

5. A method according to claims 1 and 4, characterized in that the photopolymerizable compound is applied in a plurality of layers and each layer is subjected individually to the photocuring.

6. A method according to claims 1 to 5, characterized in that in dental engineering as casting model a crown, an inlay, a bridge, the secondary part of a conical crown, telescopic crown, double crown or a male part for individual attachments an a telescoping anchor or a bar attachment or a soldering connection is built up.

## Revendications

1. Procédé pour produire des pièces métalliques coulées individuelles, au moyen d'un modèle de coulée, procédé dans lequel on
fixe au moins un canal de coulée au modèle de coulée,
noie le modèle de coulée dans une masse de moulage réfractaire,
élimine par chauffage le modèle de coulée, sans aucun résidu,
déverse un métal liquide dans le moule ainsi obtenu,
laisse refroidir ce moule et
désinsère la pièce coulée,
caractérisé par le fait qu'on réalise le modèle de coulée au moins partiellement par photopolymérisation d'une masse photopolymérisable, renfermant un ester d'acide (méth)acrylique di- ou polyfonctionnel.

2. Procédé selon la revendication 1, caractérisé par le fait que la photopolymérisation est effectuée dans la plage de longueurs d'ondes comprise entre 400 et 500 nm.

3. Procédé selon la revendication 2, caractérisé par le fait que la quinone de camphre est utilisée en tant que photoinitiateur pour la photopolymérisation.

4. Procédé selon les revendications 1 à 3, caractérisé par le fait que la masse photopolymérisable renferme des pigments organiques et/ou des opacifiants.

5. Procédé selon les revendications 1 à 4, caractérisé par le fait que la masse photopolymérisable est déposée en plusieurs couches, et chaque couche est soumise individuellement au photodurcissement.

6. Procédé selon les revendications 1 à 5, caractérisé par le fait qu'on façonne en tant que modèle de coulée, dans les techniques dentaires, une couronne, un inlay, un bridge, la partie secondaire d'une couronne conique, couronne télescopique, couronne double ou un poinçon pour systèmes télescopiques individuels, ainsi qu'un ancrage télescopique ou un chapeau d'entretoisement ou une liaison brasée.